# EUROPEAN PATENT APPLICATION

(11) **EP 4 702 968 A1**
(43) Date of publication of application: **04.03.2026**
(21) Application number: 25197562.9
(22) Date of filing: 22.08.2025
(51) Int. Cl.: A61K 8/36, A61K 8/64, A61Q 19/02

(54) **OVOTRANSFERRIN AS DEPIGMENTING AGENT IN CASES OF SKIN DYSCHROMIA/HYPERPIGMENTATION AND FORMULATIONS CONTAINING IT**

(30) Priority: 30.08.2024 IT 202400019453
(71) Applicant: Baggio, Maria Carla, 23100 Sondrio (IT)
(72) Inventor: Baggio, Maria Carla, 23100 Sondrio (IT)
(74) Representative: Bianchetti & Minoja with Trevisan & Cuonzo IPS SRL

(57) **Abstract**

A treatment method for skin dyschromia and/or hyperpigmentation of various origins and locations is described, comprising the topical application of ovotransferrin or formulations containing it. Ovotransferrin is able to significantly reduce melanin production in melanocytes.

## Description

The invention relates to formulations comprising ovotransferrin, useful for the treatment of skin dyschromia/hyperpigmentation of various origins and locations. Ovotransferrin is able to reduce melanin production in melanocytes.

### Background of the invention

Skin whitening, also known as skin lightening and bleaching, is a technique that employs agents capable of reducing skin hyperpigmentation/dyschromia (of various origins and locations) or of conferring a uniform colour by decreasing the concentration of melanin in the skin. Melanocytes function to protect the epidermis from ultraviolet radiation-induced damage through the production of melanin. Keratinocytes, the main constituents of the epidermis, progressively migrate towards the outermost layers of the skin as they undergo differentiation. Their nuclei thus become increasingly more exposed to external radiation. Melanin is transferred to the surrounding keratinocytes via the dendrites of melanocytes, in response to exposure to UV radiation or other predisposing factors and, by positioning itself above the nuclei of the keratinocytes, the melanin granules absorb the UV radiation.

Changes in pigmentation represent important conditions with potential clinical and aesthetic implications. When the production of melanin in the skin is altered, disorders such as vitiligo (doi: 10.7759/cureus.29307) and melasma (doi: 10.1007/s13555-017-0194-1) may occur. Vitiligo is the most widespread pigmentation disorder and is characterized by a loss of function of melanocytes, resulting in the appearance of light patches on the skin. On the contrary, the abnormal accumulation of melanin can lead to hyperchromias such as melasma, ephelides and freckles. The appearance of dark areas and spots on the skin may also result from the normal process of skin ageing (senile lentigo), hormonal changes (for example during pregnancy or menopause) or inflammatory acne lesions (post-inflammatory hyperpigmentation).

The accumulation of reactive oxygen species (ROS) resulting both from normal metabolism and from extrinsic factors (such as sun exposure, environmental pollution, lifestyle and habits such as smoking, hormonal alterations) can exacerbate the activity of several enzymes, such as tyrosinase, which is the main enzyme involved in melanin synthesis (doi.org/10.1016/j.jdermsci.2010.03.003). Hyperchromia caused by an increased exposure to UV, predominantly appear in areas of the body exposed to solar radiation.

Several compounds that have been shown to be effective in reducing the concentration of melanin in the skin present significant side effects.

Tretinoin (retinoic acid) is used to lighten specific areas, also in combination with steroids and hydroquinone. The most frequent side effects of tretinoin are peeling, erythema, increased risk of burns and increased risk of sunburn.

Alpha-hydroxy acids, such as glycolic acid, lactic acid, glyceric acid, malic acid and citric acid, are used as skin-lightening agents. The most common side effects of these compounds are erythema, peeling, itching and burning of the skin and increased sensitivity to UV radiation.

Kojic acid is an effective skin -lightening agent and is also used in cosmetic products at concentrations ranging from 1 % to 4% by weight. The side effects of this substance include erythema, burning sensation and contact eczema.

Glutathione, widely used orally as a skin-lightening agent, can also be applied topically in cream form. Glutathione inhibits the enzyme tyrosinase and the conversion of eumelanin to pheomelanin, both produced by melanocytes.

Potassium azeloyl diglycinate, a derivative of azelaic acid, is used to treat skin spot, acne, and skin hyperpigmentation.

Ovotransferrin, also known as conalbumin (CAS registry number 1391-06-6), is a glycoprotein with a molecular weight of 76,000 daltons contained in egg white where it constitutes approximately 13% by weight of the total proteins. This protein present in egg white has been known since 1900 (Osborne, Thomas B.; Campbell, George F. Journal of the American Chemical Society (1900), 22(7), 422-50), its purification from other albumen proteins was first described in 1940 (Longworth et al., ibid. 62, 2580 (1940)), and its primary structure has been known since 1982 (J. Williams et al., Eur. J. Biochem. 122, 297 (1982); W.-M. Keung et al., J. Biol. Chem. 257, 1177, 1184 (1982)).

Ovotransferrin has antibacterial properties (P. Valenti et al., Antimicrob. Ag. Chemother. 21, 840 (1982)), antiviral properties (F. Giansanti et al. Biochem. Biophys. Res Comm. 331, (2005), 69-73) and, due to its iron-binding properties, is being evaluated as an iron supplementation agent in humans (WO2021175509).

CN 117 209 559 describes two peptides of three or four amino acids with tyrosinase-inhibiting activity derived from the full sequence of 685 amino acids of ovotransferrin.

By virtue of its ability to bind iron, ovotransferrin is classified within the "transferrin" family (Giansanti et al., (2015), Nutrients, 7(11), 9105-9115). It shows a high structural and functional homology with other proteins of the same family, such as serum transferrin and bovine lactoferrin. Transferrins display completely distinct characteristics from those of the other proteins present in egg white, such as ovalbumin and lysozyme.

Ovotransferrin also has strong antioxidant activity (doi: 10.3382/ps.2012-02150; 10.1016/j.ijbiomac.2007.08.005), anti-inflammatory activity (https://doi.org/10.1021/acs.jafc.3c00950), and properties inhibiting the degradation of collagen and elastin of the skin (WO2001087292).

There are on the market cosmetic products with skin-lightening/depigmenting activity that contain egg whites and active ingredients with whitening activity (*Sophora flavescens* root extract, potassium azeloyl diglycinate) and exfoliating activity (salicylic acid). The skin-lightening/depigmenting effect cannot therefore be attributed to the egg white in which ovotransferrin is indeed present, but in a mixture with numerous other proteins including ovalbumin, ovomucoid, ovomucin, lysozyme and avidin, as well as many other compounds.

Albumen is included among the ingredients permitted for cosmetic use and as such, included in the European COSING database (https://single-market-economy.ec.europa.eu/sectors/cosmetics/cosmetic-ingredient-database_en) which recognises the use thereof as:
- film-forming agent on hair or skin, and as such capable of enabling flexible, cohesive and continuous coverage on hair or skin when applied to their surface
- hair conditioner, as a product that serves to improve the combability of hair, untangle knots and give hair overall protection
- cutaneous conditioner.

Medical and cosmetic research is constantly seeking new active compounds, preferably of natural origin, against skin hyperpigmentation/dyschromia that have a higher safety profile.

### Description of the Invention

It has surprisingly been found that ovotransferrin possesses skin depigmenting properties when applied topically.

A first object of the invention therefore consists of a cosmetic treatment method for skin dyschromia and/or hyperpigmentation of various origins and locations, comprising the topical application of ovotransferrin or formulations containing it.

Examples of skin dyschromia and/or hyperpigmentation comprise melasma, ephelides, freckles, senile lentigo, hormonal changes, post-inflammatory hyperpigmentation including acne-related.

The concentration of ovotransferrin can vary within wide limits, preferably from 1 to 10% by weight. Lower concentrations, for example 0.5% by weight, may be used for the treatment of skin blemishes in sensitive areas of the body such as the face.

Examples of other active ingredients that may be advantageously associated with ovotransferrin comprise UV filters, exfoliating, moisturizing, nourishing, and emollient agents.

Examples of exfoliating agents comprise glycolic, salicylic, mandelic, lactic or phytic acids.

A second object of the invention consists of topical formulations comprising as active ingredient an effective concentration of ovotransferrin, glycolic acid in concentration up to 10% by weight, mandelic acid in concentration up to 15% by weight, lactic acid in concentration up to 10% by weight and phytic acid in concentration up to 5% by weight.

Examples of UV filters comprise 4-aminobenzoic acid up to a concentration of 5% by weight, benzophenone-3 in concentration up to 10%, zinc oxide.

The formulations employed in the method of the invention (pharmaceutical, cosmetic or medical devices) may also contain other substances with plumping activity or stimulating skin regeneration for a rejuvenating effect on the skin, such as hyaluronic acid or its salts and collagen.

The optimum pH value of the formulations of the invention is between 6 and 8.

The formulations of the invention can be in the form of cream, ointment, emulsion, foam, paste, skin patch, solution, spray, skin pencil, serum, gel.

The depigmenting activity of ovotransferrin was highlighted using murine melanoma cells (B16), a well-known cell line commonly used in melanogenesis studies. In this model, ovotransferrin is able to reduce melanin levels in melanocytes by 13.0% and 9.5% compared to untreated cells, respectively at concentrations of 10.0 and 5.0 mg/ml, as reported in the following experimental part.

The invention further relates to the use of ovotransferrin as a depigmenting agent of the skin in case of skin dyschromia and/or hyperpigmentation of various origins and locations and a cosmetic treatment method of depigmentation comprising the topical application of ovotransferrin or formulations containing it.

### Experimental part

A culture of melanocytes (B16 murine melanoma cells) was used to evaluate the depigmenting action of ovotransferrin. A cell viability assay was first performed up to a maximum concentration of 20 mg/ml in order to identify the concentrations on which to conduct the subsequent test. All concentrations (including the highest concentration) were shown not to inhibit cell viability. It was thus decided to perform the depigmentation test at concentrations of 10.0, 5.0 and 1.0 mg/ml. For the depigmenting test, melanocytes were first stimulated to produce melanin and then treated with the test product at the established concentrations. One set of cells was stimulated but not treated (NC, negative control), while a further set was stimulated and treated with hydroquinone, a well-known depigmenting agent, at a concentration of 0.1 mg/ml (PC, positive control).

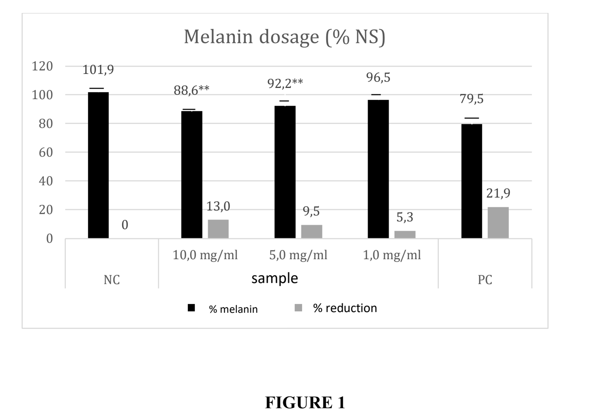

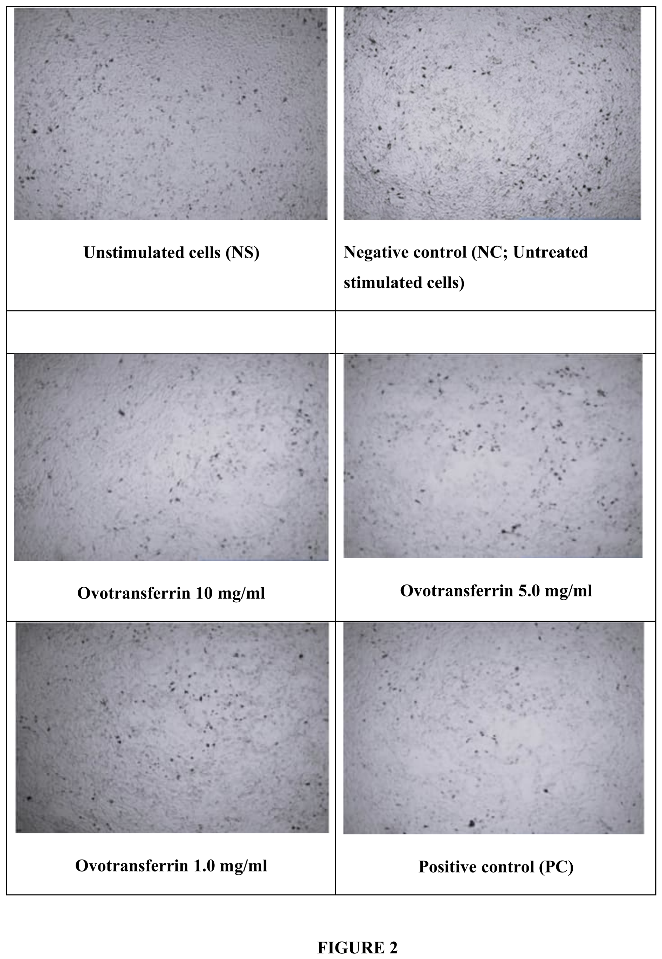

From the results obtained (Figure 1), it was found that ovotransferrin significantly reduces melanin levels in melanocytes at concentrations of 10.0 and 5.0 mg/ml. A morphological evaluation was also carried out by means of brightfield microscope photographs (Figure 2), which confirmed the quantitative data of the melanin dosage.

### Materials and Methods

Cell cultures: The test was conducted on murine melanoma cells (B16), a well-known line commonly used in melanogenesis studies. The cells were cultured in DMEM (Dulbecco's Modified Eagle Medium) containing 2 mM glutamine, 10% foetal bovine serum (FBS) and 1% antibiotics (penicillin and streptomycin) and incubated under standard culture conditions (37°C, 5% CO₂).

Sample preparation: The ovotransferrin sample is soluble in the culture medium at the concentration of 20.0 mg/ml. Therefore, to assess pre-test cell viability, the cells were treated with the sample starting from the concentration of 20.0 mg/ml and subsequent 1:3 dilutions.

### Assessment of cell viability

A cell viability assay was performed prior to melanin dosing, in order to choose the concentrations on which to perform the subsequent dosing. Cell viability was assessed by MTT test [3-(4,5-dimethylthiazol-2-yl)-2,5-diphenyltetrazolium bromide], a yellow tetrazole dye that is reduced by cells to an insoluble formazan of purple colour. This conversion is caused by NADPH or NADH produced by the dehydrogenases present in metabolically active cells. The cells were treated with MTT (1 mg/ml) and incubated for 3 hours under standard conditions. At the end of this period, the MTT solution was removed and 100 µl of isopropanol was added to each well to dissolve the formed formazan crystals. Absorbance (optical density, OD) was determined by spectrophotometer reading at a wavelength of 570 nm.

Melanin dosage: The B16 cells were plated and kept constantly in the dark under standard culture conditions. The negative control was represented by untreated cells, while hydroquinone, a substance with a known depigmenting action, was used as a positive control (PC). For the melanin content analysis, the cells were collected and lysed. Melanin was quantified with a spectrophotometer at the wavelength of 490 nm. Just prior to cell lysis, the melanocytes were observed and photographed in bright field microscopy.

### Preliminary assessment of cell viability

The absorbance (OD) measured at 570 nm is proportional to cell viability. The percentages reported in the table were calculated based on the OD values at 570 nm and considering the OD of the untreated cells as 100% (Table 1).

**Table 1**

| | **NC** | **ovotransferrin concentration (mg/ml)** | | | | | | | |
|---|---|---|---|---|---|---|---|---|---|
| | | 0.0091 | 0.0274 | 0.082 | 0.25 | 0.7 | 2.2 | 6.7 | 20.0 |
| **Average OD** | 2.305 | 2.346 | 2.313 | 2.281 | 2.261 | 2.266 | 2.315 | 2.305 | 2.249 |
| **Viability (%)** | 100.0 | 101.7 | 100.3 | 99.0 | 98.1 | 98.3 | 100.4 | 100.0 | 97.5 |
| NC= negative control | | | | | | | | | |

According to the results obtained, the test for the evaluation of depigmenting activity was carried out using the concentrations of 10.0, 5.0 and 1.0 mg/ml of ovotransferrin (Table 2; Figure 1).

### Melanin dosage after 48 hours

**Table 2**

| | **NC** | **ovotransferrin** | | | **PC** |
|---|---|---|---|---|---|
| | | **10.0 mg/ml** | **5.0 mg/ml** | **1.0 mg/ml** | |
| **% melanin** | 101.9 | 88.6 | 92.2 | 96.5 | 79.5 |
| **% reduction** | n.a. | 13.0 | 9.5 | 5.3 | 21.9 |
| NC= negative control; PC= positive control; n.a.=not applicable | | | | | |

The absorbance measured at 490 nm is directly proportional to the amount of melanin extracted from the cells. The percentages are calculated with respect to the melanin levels of unstimulated cells (NS). The values are expressed as mean ± standard deviation. The data was statistically processed by means of Student's t-test. P-values<0.05 are considered significant. NS = unstimulated cells; NC = untreated stimulated control cells; PC = positive control (stimulated cells treated with hydroquinone); sample = stimulated cells treated with ovotransferrin at different concentrations. **p<0.01 vs NC

Ovotransferrin significantly (p<0.01) reduces melanin production at concentrations of 10.0 and 5.0 mg/ml (reduction of 13.0% and 9.5%, respectively, with respect to the untreated cells). Figure 1.

Cells treated with ovotransferrin at concentrations of 10.0 and 5.0 mg/ml appear less dark due to the lower presence of melanin.

The results obtained demonstrate that ovotransferrin has a marked depigmenting effect and highly significantly (p<0.01) reduces the amount of melanin in melanocyte cultures.

The quantitative compositions of formulations of the invention are given in the following examples.

### Example 1 ointment

| **Components** | **% (w/w)** |
|---|---|
| Purified water | Q. s. to 100 |
| Sodium hydroxide 30% | Q. s. to pH 7.0 |
| Propylparaben | 0.02 |
| Sodium Lauryl Sulphate | 1.05 |
| Propylene Glycol | 12.6 |
| Stearyl Alcohol | 26.3 |
| Methylparaben | 0.03 |
| Ovotransferrin | 1.0 |
| Glycolic Acid | 10.0 |
| Benzophenone-3 | 6.0 |
| White Vaseline | 26.32 |

### Example 2 solution

| **Components** | **% (w/w)** |
|---|---|
| Purified water | Q. s. to 100 |
| Sodium hydroxide 30% | Q. s. to pH 7.0 |
| EDTA disodium salt | 0.2 |
| Ovotransferrin | 1.0 |
| Glycerol | 10.0 |
| Glycolic Acid | 10.0 |
| Sepineo^{™} d.e.r.m. | 2.0 |
| Benzophenone-3 | 6.0 |

### Example 3 cream

| **Components** | **% (w/w)** |
|---|---|
| Purified water | Q. s. to 100 |
| Sodium hydroxide 30% | Q. s. to pH 7.0 |
| Liquid paraffin | 10.0 |
| Ovotransferrin | 1.0 |
| Propylene Glycol | 5.0 |
| Glycolic Acid | 10.0 |
| Glycerine | 10.0 |
| Polysorbate 60 | 1.3 |
| Benzophenone-3 | 6.0 |

### Example 4 gel

| **Components** | **% (w/w)** |
|---|---|
| Ethanol | 30.8 |
| Ovotransferrin | 1.0 |
| Sodium hydroxide 30% | Q. s. to pH 7.0 |
| Propylene Glycol | 13.9 |
| Glycolic Acid | 10.0 |
| Purified water | Q. s. to 100 |
| Diethyleneglycol monoethyl ether | 3.5 |
| Myristic alcohol | 2.1 |
| Benzophenone-3 | 6.0 |

### Example 5 serum:

| **Components** | **% (w/w)** |
|---|---|
| Purified water | Q. s. to 100 mL |
| Natural Synergy Preservative System (INCI Caprylhydroxamic Acid (and) Propanediol, Glyceryl Caprylate). | 1 |
| Siligel^{™} (INCI: Xanthan Gum, Lecithin, Sclerotium Gum, Pullulan). | 1 |
| Ovotransferrin | 1 |

### Example 6 foam

| **Ingredient** | **INCI** | **% without propellant** | **% with propellant** |
|---|---|---|---|
| Ovotransferrin | | 1% | 90 |
| Glycerine | | 3% | |
| Olivoyl glutamate emulsifier^{®} | olivoyl glutamate (25-50%), cetearyl alcohol (25-50%), glyceryl stearate (25-50%), potassium sorbate <1%, sodium benzoate <1%, benzyl alcohol <1% | 6% | |
| Protelan agl 95 | Sodium lauroyl glutamate | 0.5% | |
| Euxyl^{™} k 712 | Water, sodium benzoate, potassium sorbate | 0,700% | |
| Vitamin E acetate | | 0.3%% | |
| Jojoba oil | | 3 | |
| Water | | as needed 100 | |
| n-butane propane isobutane pressure 3.2 Psi | | - | 10 |

## Claims

1. Treatment method for skin dyschromia and/or hyperpigmentation of various origins and locations comprising the topical application of ovotransferrin or formulations containing it.

2. Method according to claim 1, wherein ovotransferrin is applied to the skin in a concentration from 0.5 to 10% by weight.

3. Method according to claim 1 or 2, wherein ovotransferrin is associated with other active ingredients selected from UV filters, exfoliating agents, moisturizers, nutrients, emollients, hyaluronic acid or its salts, collagen.

4. Method according to claim 3, wherein the exfoliating agents comprise glycolic, salicylic, mandelic, lactic or phytic acids.

5. Method according to any one of claims 1-4, wherein UV filters are selected from 4-aminobenzoic acid, benzophenone-3, zinc oxide.

6. Ovotransferrin for use in the pharmacological treatment of skin dyschromia and/or hyperpigmentation of various origins and locations.

7. Topical formulations comprising as active ingredient an effective concentration of ovotransferrin, glycolic acid in concentration up to 10% by weight, salicylic acid in concentration up to 2% by weight, mandelic acid in concentration up to 15% by weight, lactic acid in concentration up to 10% by weight and phytic acid in concentration up to 5% by weight.

8. Formulations according to claim 7, **characterized by** pH 6-8.

9. Formulations according to claim 7 or 8 in the form of cream, ointment, emulsion, foam, paste, skin patch, solution, spray, skin pencil, serum, gel.
